# EUROPEAN PATENT SPECIFICATION

(11) **EP 3 215 019 B1**
(45) Date of publication and mention of the grant of the patent: **15.05.2019**
(21) Application number: 15817697.4
(22) Date of filing: 10.12.2015
(51) Int. Cl.: A61B 8/12, A61B 8/08

(54) **IMAGING DEVICE**
BILDGEBUNGSVORRICHTUNG
DISPOSITIF D'IMAGERIE

(30) Priority: 07.01.2015 US 201562100756 P
(43) Date of publication of application: 13.09.2017
(73) Proprietor: St. Jude Medical, Cardiology Division, Inc., St. Paul, MN 55117 (US)
(72) Inventor: LUPOTTI, Fermin A., Lake Forest, California 92630 (US); SLIWA, John W., San Jose, California 95110 (US); MA, Zhenyi, Santa Clara, California 95050 (US); MORSE, Stephen A., Menlo Park, California 94025 (US)
(74) Representative: Kramer Barske Schmidtchen Patentanwälte PartG mbB
(86) International application number: PCT/US2015/064960
(87) International publication number: WO 2016/111804

(56) References cited:
- US-A1- 2007 232 921
- US-A1- 2008 287 797
- US-A1- 2010 168 570
- US-A1- 2011 071 401
- US-A1- 2012 059 241
- US-A1- 2014 107 492
- US-B1- 6 485 413
- DOUGLAS M. CAVAYE ET AL: "A New Technique for Intraluminal Hollow Organ Imaging: Three-Dimensional Ultrasound", JOURNAL OF LAPAROENDOSCOPIC SURGERY, vol. 1, no. 5, 1 January 1991 (1991-01-01) , pages 259-268, XP055260500,

## Description

### BACKGROUND

The instant disclosure relates to imaging, including medical imaging. In particular, the instant disclosure relates to an apparatus for creating three-dimensional volumetric images.

Ultrasound transducers are utilized in a variety of medical applications. In many applications, the transducer is mounted in a catheter that can be navigated through a patient's vasculature and/or body organs to a site of interest.

In many such catheters, in order to obtain a three-dimensional volumetric image of the tissue being imaged, the transducer is rotated around a longitudinal axis of the catheter in order to obtain a plurality of two-dimensional image slices for assembly into a three-dimensional volumetric image. The transducer, for example a phased two dimensional image array, can be rotated via a motor or a manual actuator (*e.g*., a finger slider), either of which necessitates a relatively complex, relatively large diameter, and expensive catheter structure. For example, a motorized continuously-rotating transducer typically requires a rotating drivewire, a rotating energized ("hot") lead, and a rotating ground lead, as well as electrical slip rings or rotary transformers in or near the catheter handle.

US 2007/0232921 A1 discloses an apparatus comprising the features of the preamble of claim 1.

### BRIEF SUMMARY

The invention is defined in the appended claims. Disclosed herein is an apparatus for imaging tissue that includes: an acoustic imaging element (*e.g*., a phased array two-dimensional imaging transducer) having an active face that emits energy along a beam path and towards a tissue to be imaged; and an acoustically transmissive oscillating energy deflector positioned within the beam path. The acoustically transmissive oscillating energy deflector can be an acoustically transparent prism or lens. A drive assembly can be coupled to and operable to oscillate the acoustically transmissive energy deflector. For example, the drive assembly can include a motor and/or piezomotor, a cyclically inflatable element, and/or can be a cyclically fluid-driven assembly. Oscillating the deflector allows the capture of multiple closely-spaced and/or overlapping two-dimensional image slices that can be assembled to create a three-dimensional volumetric image.

In certain embodiments, the acoustically transmissive energy deflector oscillates at a frequency of between 15 Hz and 30 Hz, allowing for the capture of between 30 and 60 volumes per second (*e.g*., one volume in each direction of the oscillation). It can also oscillate through a range of 70 degrees.

It is contemplated that the acoustic imaging element and the acoustically transmissive energy deflector can be disposed within an enclosure, such as the catheter shaft or an inflatable balloon or membrane.

Also disclosed herein is an apparatus for imaging tissue including: an acoustic imaging element (*e.g*., a phased array two-dimensional imaging transducer) having an active face that emits energy along a beam path and towards a tissue to be imaged; and an oscillating reflective acoustic mirror deflector positioned within the beam path. The reflective acoustic mirror can be secured to the apparatus via at least one elastic element biased such that, when the elastic element is in a relaxed position, the acoustic mirror forms an angle of zero degrees with the active face of the acoustic imaging element (*e.g*., it lays flat against the apparatus/imaging element). A drive assembly, including one or more of a cyclically inflatable element, a motor, and a piezomotor, can be coupled to and operable to oscillate the acoustic mirror.

An apparatus for volumetrically imaging tissue includes: a shaft; an imaging element disposed within the shaft, the imaging element including an active face that emits energy along a beam path and towards a tissue to be imaged; an energy deflector (*e.g*., a prism, lens, or acoustic mirror) positioned within the beam path; and a drive assembly coupled to the energy deflector operable to oscillate the energy deflector. The apparatus can also include a sensor for measuring a rotational or deflected position of the energy deflector as it oscillates. For example, the drive assembly can include a stepper motor.

The apparatus can also include a processor to assemble a three-dimensional volumetric image of the tissue to be imaged from a plurality of two-dimensional image slices of the tissue, wherein each image slice of the plurality of two-dimensional image slices is associated with a corresponding rotational or deflected position of the energy deflector. It is contemplated that the processor can also include additional functions, such as graphical user interface ("GUI") presentation, system control, deflection control, and the like.

The imaging element will emit energy along the beam path to form two-dimensional image slices at a frame rate, and the energy deflector will oscillate at an oscillation frequency. It is contemplated that the frame rate and the oscillation frequency will be integer multiples of each other, and can be identical (*e.g*., the integer can be 1).

It should be understood from the foregoing summary and the detailed description that follows that, to form a three-dimensional volumetric image, the imaging element (*e.g*., a phased array two-dimensional imaging transducer) can have its electronically-scanned two-dimensional image plane mechanically deflected in a deflection direction, which is out of or at an angle to the imaging element's own two-dimensional image plane. This allows a set of closely spaced and/or overlapping two-dimensional image slices to be acquired, which, when assembled, create the three-dimensional volumetric image.

It should also be understood from the foregoing summary and the detailed description that follows that the two-dimensional image slices may not be perfectly parallel to each other in space, for example if the deflection mechanism swings the energy deflector about a hinge or pivot axis. Thus, as used herein, the term "deflection" (and its derivatives, such as "deflect" or "deflector") includes not just pure rotation, but also a combination of rotation and translation. The various processors described herein can also compensate for any non-parallel offset in two-dimensional image slices.

The foregoing and other aspects, features, details, utilities, and advantages of the present invention will be apparent from reading the following description and claims, and from reviewing the accompanying drawings.

### BRIEF DESCRIPTION OF THE DRAWINGS

Figure 1 is a representative intracardiac echocardiography ("ICE") catheter.
Figure 2 is a close-up and partially cut-away view of the distal section of the catheter depicted in Figure 1.
Figure 3a is an axial cross-section of a first embodiment of an imaging device as disclosed herein taken along line 3-3 in Figure 2.
Figure 3b is an axial cross-section of a second embodiment of an imaging device as disclosed herein taken along line 3-3 in Figure 2.
Figure 3c is an axial cross-section of a third embodiment of an imaging device as disclosed herein taken along line 3-3 in Figure 2.
Figure 3d is an axial cross-section of a fourth embodiment of an imaging device as disclosed herein taken along line 3-3 in Figure 2.
Figure 4 defines an angle α for the oscillation of an energy deflector 24 according to embodiments disclosed herein.
Figure 5 is a block diagram of a system to construct a three-dimensional volumetric image according to aspects of the teachings herein.
Figure 6 is a graphical representation of the use of the imaging devices disclosed herein to capture a plurality of two-dimensional image slices for assembly into a three-dimensional volumetric image.
Figure 7 illustrates an imaging element fitted with an asymmetric lens.
Figure 8a is a close-up and cut-away view of the distal end of an ICE catheter including an imaging element fitted with an asymmetric lens, such as shown in Figure 7.
Figures 8b-8d are axial cross-sections of Figure 8a taken along lines b-b, c-c, and d-d, respectively.
Figure 9 illustrates an embodiment of an ICE catheter including an oscillating acoustic mirror according to the teachings herein.
Figure 10 illustrates an alternative construction of an ICE catheter including an oscillating acoustic mirror as disclosed herein.
Figure 11 illustrates the use of an inflatable balloon to encapsulate the imaging elements and energy deflectors disclosed herein.

### DETAILED DESCRIPTION

The present disclosure provides three dimensional imaging apparatuses, systems, and methods. For purposes of illustration, certain exemplary embodiments will be described herein in the context of an intracardiac echocardiography ("ICE") device, such as the ViewFlex™ Xtra ICE Catheter of St. Jude Medical, Inc. It is contemplated, however, that the apparatuses, systems, and methods described herein can be used in other contexts, including, without limitation, intravascular ultrasound ("IVUS") devices.

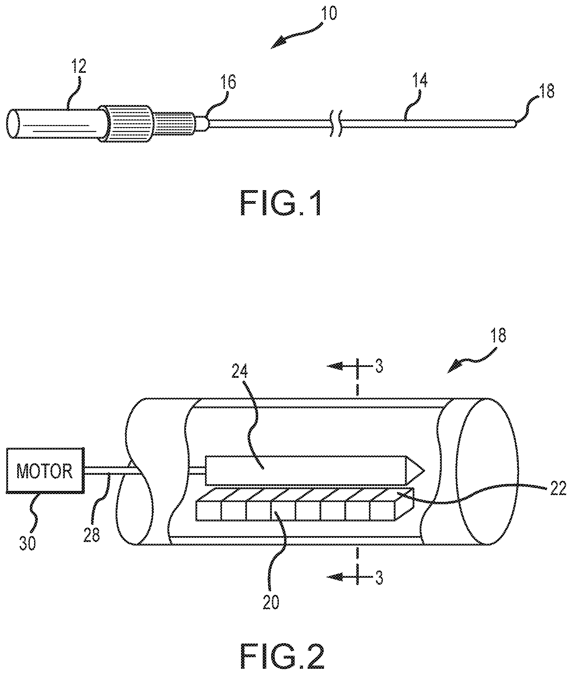

Figure 1 depicts a representative ICE catheter 10. ICE catheter 10 generally includes a handle 12 and a shaft 14, which has a proximal section 16 (connected to handle 12) and a distal section 18. The basic construction and features of ICE catheter 10 (*e.g*., steerability of distal section 18) will be familiar to the ordinarily skilled artisan. Thus, in the interest of brevity, the features of ICE catheter 10 will only be described in detail herein to the extent necessary to understand the instant disclosure.

As shown in the close up and partially cut-away view of Figure 2, distal section 18 of shaft 14 includes therein an imaging element 20. Imaging element 20 includes an active face 22 that emits and receives energy along a beam path and towards a tissue to be imaged (*e.g*., a cardiac surface). Distal section 18 can be somewhat larger in diameter than proximal section 16 in order to accommodate imaging element 20 and the other aspects of the disclosure described below.

In the case of ICE catheter 10, imaging element 20 is an acoustic element, and more particularly an ultrasound element. For example, imaging element 20 can be a multi-element (*e.g*., 64 element) phased or linear ultrasound two-dimensional transducer array or any other suitable ultrasound transducer (including a single-element transducer) or arrangement of multiple ultrasound transducers (each of which can, for example, be either single- or multi-element). It should be understood, however, that in embodiments not forming part of the present invention any suitable imaging element can be employed, including both acoustic and/or electromagnetic (*e.g*., optical, near-infrared) elements. In general, the ordinarily skilled artisan will appreciate how to select and configure a suitable imaging element 20 for a given application of the teachings herein.

Distal section 18 also includes an energy deflector 24 positioned in the beam path of imaging element 20. Energy deflector 24 acts to deflect, steer, shape, focus, defocus, or otherwise alter the energy emitted by imaging element 20 as it passes therethrough (in the case of an acoustic prism or acoustic lens) or reflects therefrom (in the case of an acoustic mirror). The ordinarily skilled artisan will understand from the foregoing disclosure that imaging element 20 emits energy towards the tissue being imaged through (*e.g*., in the case of a lens or prism) or off of (*e.g.,* in the case of an acoustic mirror) energy deflector 24, which redirects the energy as it propagates.

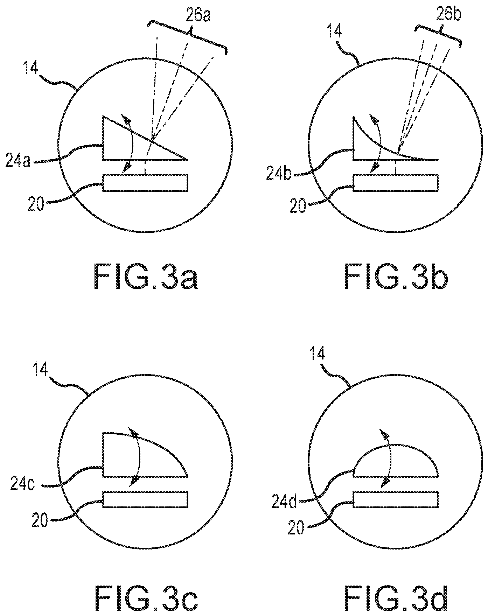

Figures 3a through 3d depict, in axial cross-section, various embodiments of energy deflector 24. In a first embodiment, depicted in Figure 3a, energy deflector 24 is a transmissive prism 24a. In another embodiment, depicted in Figure 3b, energy deflector 24 is a transmissive lens or lensed prism 24b. The use of a transmissive lens or lensed prism 24b allows the energy 26b to maintain a tighter pattern after passing through and being deflected by energy deflector 24 than is the case with energy 26a passing through ordinary transmissive prism 24a in Figure 3a. That is, the transmissive lens or lensed prism 24b results in a thinner (*i.e.,* more collimated) imaging plane or beam width after the beam passes therethrough. Figure 3c depicts an alternative configuration of a transmissive lens or lensed prism 24c. As used herein, the term "transmissive" means deflection element 24 has beam energy passing through its bulk in at least one direction. That passage may involve a single pass (e.g., as shown in Figures 3a and 3b) or multiple passes involving an internal reflection within deflection element 24. In general, the person of ordinary skill in the art will be familiar with the principles relevant to the selection, configuration, and/or design of acoustic prisms and acoustic lenses, such as 24a, 24b, and 24c, given the nature and purpose of the device within which the same is installed, the corresponding configuration and purpose of imaging element 20 (*e.g*., the number and arrangement of transducers and/or the number and arrangement of elements within transducers), and the like. For example, certain design considerations for acoustic prisms are discussed in Li et al., Unidirectional acoustic transmission through a prism with near-zero refractive index, Appl. Phys. Lett. 103, 053505 (2013).

Those of ordinary skill in the art will appreciate that imaging element 20 will not only emit energy through (or off of) deflection element 24, but will also receive incoming acoustic energy through (or off of) deflection element 24. Thus, it is desirable to design distal section 18 (*e.g*., imaging element 20, deflection element 24, and the like) to reduce multiple internal reflections and reverberations in or off of deflection element 24. It is also desirable to ensure that any gaps (*e.g*., the varying-size gap between imaging element 20 and deflection element 24 as deflection element 24 oscillates) are filled with an acoustically-transmissive liquid or other flowable material that minimizes acoustic reflections due to acoustic impedance mismatches. For example, as discussed below, one such flowable material is saline. In other embodiments, however, a permanent gel can be used to fill the gaps as they vary.

Figure 3d shows yet another alternative embodiment where energy deflector 24 is a non-prismatic lens 24d.

It should be understood, however, that Figures 3a through 3d are exemplary embodiments of acoustically transmissive energy deflectors 24 according to the teachings herein. The ordinarily skilled artisan will appreciate that other configurations of energy deflector 24 can be used, depending upon the device within which energy deflector 24 is installed, the intended use therefor, the nature and configuration of imaging element 20, and the material of which energy deflector 24 is made (although an acoustically transmissive deflection element 24 will generally be made of a material having low acoustic attenuation and an acoustic impedance not very different from surrounding saline). For example, suitable materials for the acoustic prisms described herein include, without limitation, metamaterials as described by Li et al. (cited above), phononic crystals, TPX, Upilex, and silicone rubber.

The ordinarily skilled artisan will also understand that shaft 14 can be filled with a medium (*e.g*., saline, as described above) in order to facilitate the transmission of ultrasonic energy emitted by imaging element 20 as it propagates towards, off of, and/or through energy deflector 24. Advantageously, saline acts as an acoustic coupling material to reduce loss/reflection of acoustic energy at the transducer interface.

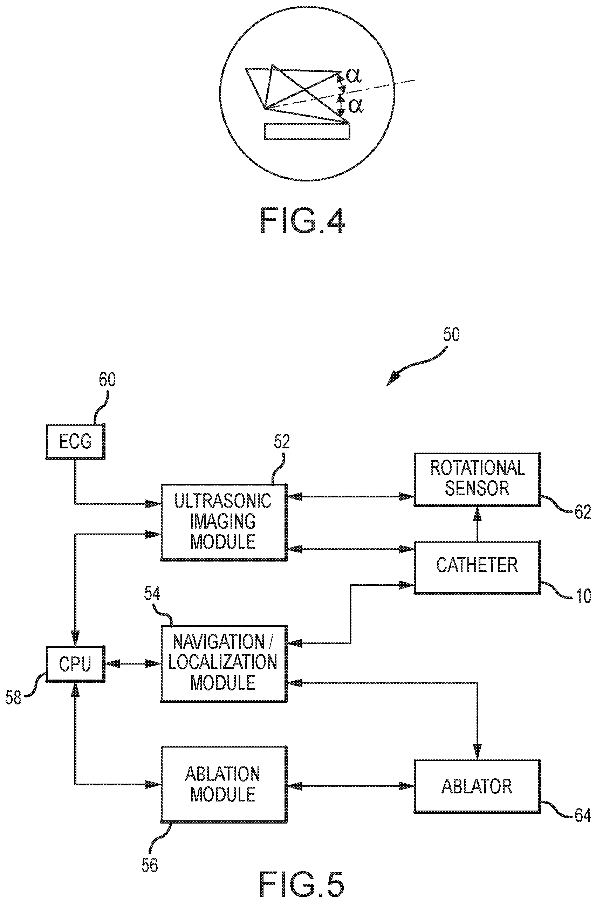

It will be understood from the description herein that, for each rotational position of energy deflector 24 relative to the longitudinal axis of shaft 14 (*see* the arrows in Figures 3a-3d and 4), imaging element 20 will be able to capture a corresponding two-dimensional image slice of the tissue to be imaged. It will further be understood that a plurality of two-dimensional image slices, corresponding to a plurality of rotational or deflected positions of energy deflector 24 as it rotates, oscillates, and/or deflects, can be assembled to produce a three-dimensional volumetric image of the tissue to be imaged as discussed in further detail below.

Rather than rotating the entirety of catheter 10 to capture various rotational orientations as is the case in some prior art devices, and rather than rotating imaging element 20 within catheter 10, which introduces additional complexity to the construction of catheter 10, as is the case in other extant devices, energy deflector 24 can be rotated by itself to capture a plurality of two-dimensional image slices that collectively define a three-dimensional volumetric image. More particularly, energy deflector 24 can be oscillated about the longitudinal axis of shaft 14 (*e.g.,* on a hinge or pivot that runs parallel to the longitudinal axis of shaft 14); as energy deflector 24 oscillates, the energy passing therethrough (or reflected therefrom, in the case of an acoustic mirror, as described below) will impinge upon a different slice or portion of the tissue to be imaged.

The pivot or hinge about which energy deflector 24 rotates or oscillates may be positioned running through energy deflector 24, on a side of energy deflector 24, or elsewhere. For example, in some embodiments, a drive shaft (or drive wire) 28 can be attached to energy deflector 24 at one end and to a motor 30 (shown schematically in Figure 2) at its other end. Motor 30 can be engaged to oscillate or position drive shaft 28, and therefore energy deflector 24, back and forth. Alternatively, motor 30 can be engaged to rotate drive shaft 28, with suitable mechanisms used to convert the rotational motion of drive shaft 28 into oscillatory motion of energy deflector 24 in distal section 18.

In some embodiments, motor 30 can be a piezomotor (*e.g*., a rotary piezomotor), which may be situated in handle 12 and connected by drive shaft 28 to energy deflector 24. In other embodiments, the piezomotor can be disposed within distal section 18, which advantageously simplifies construction by reducing or eliminating the need for drive shaft 28.

Motor 30 can also be a stepper motor, a reversible stepper motor, or a servo motor.

In still other embodiments, energy deflector 24 can be rotated by an inflatable balloon or membrane, wherein inflation of the balloon or membrane forces deflection element 24 to move via contact therewith and/or mechanical coupling thereto. Such a balloon or membrane may be oscillated in inflation-extent for rotational scanning as by a fluid or gas piston residing in handle 12. The piston can be driven, for example by a linear piezoactuator, and can be fluidically connected to the driving or oscillating balloon or membrane via a pressurized fluid lumen in shaft 14. An advantage of the foregoing embodiment is that it minimizes or avoids the torque of drive shaft 28, which can cause unintended wholesale rotation of the entire distal section 18, as opposed to just the deflection element 24 as desired.

In further embodiments, drive shaft 28 may include a rotatable drive wire inside a rotationally-static tube (*e.g*., a rotatable nitinol drive wire in a rotationally-static nitinol tube). By attaching the body of motor 30 to the containment tube and the drive shaft of motor 30 to the drive wire, it is possible to minimize unwanted torque being delivered to distal section 18. The drive wire may have a lubricious coating (*e.g*., polytetrafluouroethylene (PTFE), such as TEFLON®) to minimize stick/slip events between the drive wire and the tube within which it is constrained.

As yet another alternative, energy deflector 24 can be driven as disclosed in US 2010/0168570 A1. fluid (*e.g*., saline) driving the impeller as disclosed in the foregoing patent application can also advantageously act as described above to facilitate the transmission of ultrasonic energy emitted by imaging element 20. The fluid could also cyclically drive a bellows- or balloon-type mechanism (*e.g*., as the fluid fills the balloon or bellows, α (described below) gets progressively larger; as fluid is drained therefrom, α gets progressively smaller), rather than turning an impeller.

According to additional aspects of the disclosure, energy deflector 24 is driven by a temperature-driven shape memory actuator.

According to certain aspects, energy deflector 24 oscillates through a range of about 70 degrees (*e.g.,* ± α, as shown in Figure 4, are each about 35 degrees from the "neutral" position, which is shown by a dashed line). Of course, it is within the scope of the instant teachings for ± α to be other values, recognizing that greater oscillatory ranges will yield additional two-dimensional image slices, and therefore a larger (that is, including a greater number of two-dimensional image slices) three-dimensional volumetric image.

The ordinarily skilled artisan will appreciate from the present disclosure that the upper limit on the oscillation frequency of energy deflector 24 will be dictated by the speed of sound in the medium to be imaged and by excessive fluid drag/cavitation associated with any saline or liquid surrounding energy deflector 24 as it moves. In certain embodiments, however, the oscillation frequency of energy deflector 24 will be between about 15 Hz and about 30 Hz.

Indeed, it is desirable to oscillate energy deflector 24 at the frequency at which imaging element 20 emits energy (commonly referred to as the "frame rate"), or at a rate that is an integer multiple of the frame rate, to improve the efficiency with which the three-dimensional volumetric image is assembled. For example, in one embodiment, a single 3D volume is gathered by 180 degrees of the full 360 degrees of phase of the full oscillation cycle (that is, each full cycle over the angular deflection limits can provide 2 sequential volumes).

As described above, each rotational position of energy deflector 24 is associated with a corresponding two-dimensional image slice of the tissue to be imaged. Thus, for example, a two-dimensional image slice can be taken at each degree step as energy deflector 24 oscillates from, *e.g.,* -35 degrees to 35 degrees (*i.e.,* a 180 degree of phase or half cycle of a full sine wave oscillation) relative to the position designated as "neutral" for a total of 71 two-dimensional image slices. These 71 two-dimensional image slices can be assembled into a single three-dimensional volumetric image of the tissue to be imaged. Depending on the oscillation rate of energy deflector 24, multiple volumetric images can be created each second, which facilitates the smooth depiction of cardiac motion.

Figure 5 is a block diagram of a system 50 to gather a plurality of such two-dimensional image slices and then assemble a three-dimensional volumetric image therefrom. System 50 can also be employed to monitor an ablation device. As shown in Figure 5, system 50 can include an ultrasonic imaging module 52, a navigation/localization module 54, and an ablation module 56, all of which can be under control of and/or executed on a processor 58. An ECG 60 can also be provided.

In the embodiment depicted in Figure 5, catheter 10 (and, in particular, imaging element 20 thereof) is in communication with ultrasonic imaging module 52. A rotational sensor 62 is also in communication with ultrasonic imaging module 52. In particular, rotational sensor 62 measures the rotational position or deflection of energy deflector 24 and provides that information to ultrasonic imaging module 52, so that it can be associated with the corresponding two-dimensional image slice (that is, so that each two-dimensional image slice has an associated angle α and/or frame number that can be used to order or sequence the image slice when assembling the three-dimensional volumetric image). Additional rotational sensors 62 and/or position sensors (*e.g*., localization elements) can also be provided within distal section 18. This allows determination of both the deflection of energy deflector 24 relative to distal section 18 and, optionally, the spatial orientation and position of distal section 18 relative to the anatomy, which will be influenced by the practitioner and the beating heart.

As described above, in certain aspects of the disclosure, motor 30 is a stepper or servo motor, such that the various rotational positions of energy deflector 24 are known (*e.g*., by a motor-integrated encoder). Alternatively, a rotary encoder (which can be mechanical, optical, magnetic, capacitive, or of any other suitable technology) can be used at distal section 18 to output the rotational position of energy deflector 24.

Another suitable rotational sensor 62 is an electromagnetic coil. As described above, two such coils can be used, with a first mounted on energy deflector 24 and a second mounted on distal section 18 itself. This enables one to detect the rotational position of energy deflector 24, for example by driving the first coil and detecting the first coil using the second coil (*e.g*., the coil on distal section 18 itself) via mutual induction coupling. Likewise, to determine the orientation and position of distal section 18, an external magnetic field can be applied and the response of the second coil can be measured (as is generally known in connection with magnetic field-based localization systems, including those referenced herein).

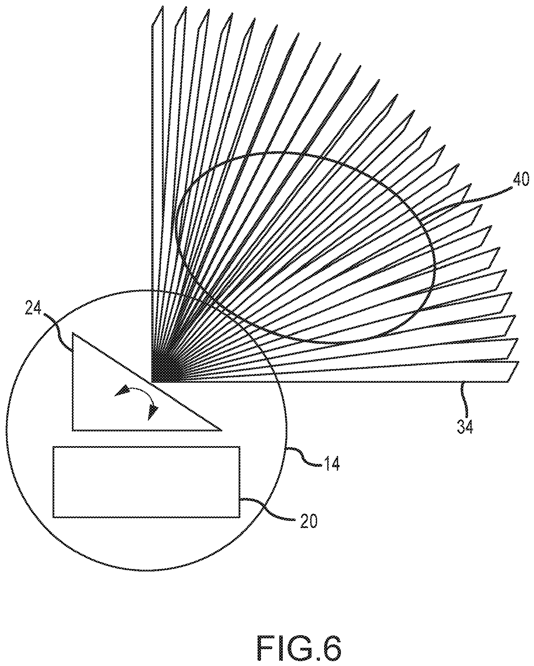

In turn, ultrasonic imaging module 52, under control of (and/or executing on) processor 58, assembles a plurality of such image slices, according to their associated rotational positions, into a volumetric image. To aid in understanding the assembly of a plurality of image slices into a volumetric image by processor 36, Figure 6 is a schematic representation of the intersection of a plurality of image slices 34 with a tissue volume to be imaged 40.

Navigation/localization module 54 is operable to detect the position, and, in some aspects, rotational orientation, of a medical device, such as catheter 10 and/or ablator 64, within a localization field. When navigation/localization module 54 also localizes catheter 10, the localization of catheter 10 can be used to identify the location of the tissue depicted in the three-dimensional volumetric image assembled as discussed above.

In some embodiments, navigation/localization module 54 is the EnSite™ Velocity™ cardiac mapping and visualization system of St. Jude Medical, Inc., which operates on the principle that, when electrical currents are passed through a resistive medium, the voltage sensed by a tracking electrode can be used to determine the positon of a medical device within the body. Other similar systems that rely upon electrical fields to localize a medical device within a patient's body can also be used. Other systems, however, may be used in connection with the present teachings, including for example, the CARTO navigation and location system of Biosense Webster, Inc., the AURORAS system of Northern Digital Inc., or Sterotaxis' NIOBE® Magnetic Navigation System, all of which utilize magnetic fields rather than electrical fields. The localization and mapping systems described in the following patents can also be used: United States Patent Nos. 6,990,370; 6,978,168; 6,947,785; 6,939,309; 6,728,562; 6,640,119; 5,983,126; and 5,697,377. Insofar as various navigation/localization systems (including those mentioned above) are well known, however, a detailed explanation thereof is not necessary to the instant disclosure.

Ablator 64 is also in communication with ablation module 56. Ablator 64 can include a radiofrequency ablation catheter and a radiofrequency generator to drive the catheter, but other manners of ablation (*e.g*., ultrasound ablation, cryogenic ablation, laser ablation) are contemplated. A detailed description of ablation module 56, however, is not necessary to the understanding of the teachings herein. Instead, it will suffice to mention that the imaging teachings herein can be applied to good advantage to observe and monitor the progress of a lesion being created by ablator 64.

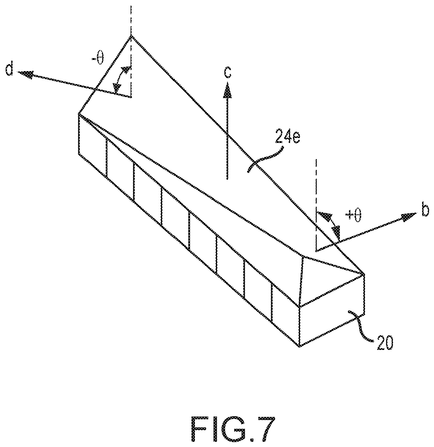

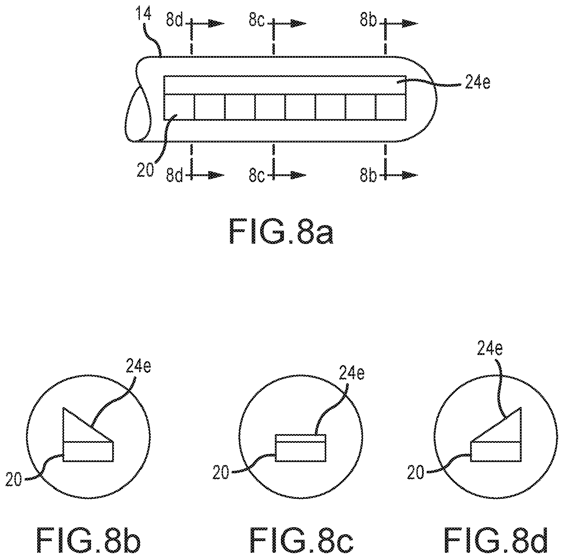

In another aspect of the disclosure, which does not form part of the present invention, energy deflector 24 does not oscillate to capture the plurality of image slices 34 that are assembled into the desired three-dimensional volumetric image. Instead, as shown in Figures 7 and 8a-8d, energy deflector 24 is an asymmetric transmissive lens 24e. An asymmetric transmissive lens 24e is twisted or warped such that, at its ends, it captures the outermost image slices (that is, those corresponding to large values of the angle θ, as measured from a line normal to the surface of imaging element 20) and, toward its center, it captures the more central image slices (that is, those where the angle θ, as measured from a line normal to the surface of imaging element 20, is close to zero).

As such, asymmetric transmissive lens 24e facilitates the capture of image slices at various rotational orientations, without mechanical oscillation, such as by sequentially activating one or more elements within imaging element 20 and "walking" the activated elements along the length of imaging element 20 (*e.g*., activating a moving window of 8 elements of a total of 64 elements within imaging element 20).

In certain embodiments, asymmetric lens 24e can capture image slices over a range of up to about 30 degrees (*e.g.,* ± about 15 degrees from the "neutral" position normal to the surface of imaging element 20, shown in Figure 8c). Arrows b, c, and d in Figure 7 show the direction in which the image slice is taken at various points along asymmetric prism 24e.

The image slices can be gathered by sequentially activating different elements (or subsets of elements) within imaging element 20. Each activation can be termed an "aperture," and, by "walking" the aperture along imaging element 20, the plurality of two-dimensional image slices can be captured. Advantageously, this avoids any mechanical oscillation of or within distal section 18.

It should also be understood, by analogy to Figures 3a-3d and their corresponding description, that asymmetric prism 24e could be replaced or supplemented with various configurations of an asymmetric lens to achieve similar results.

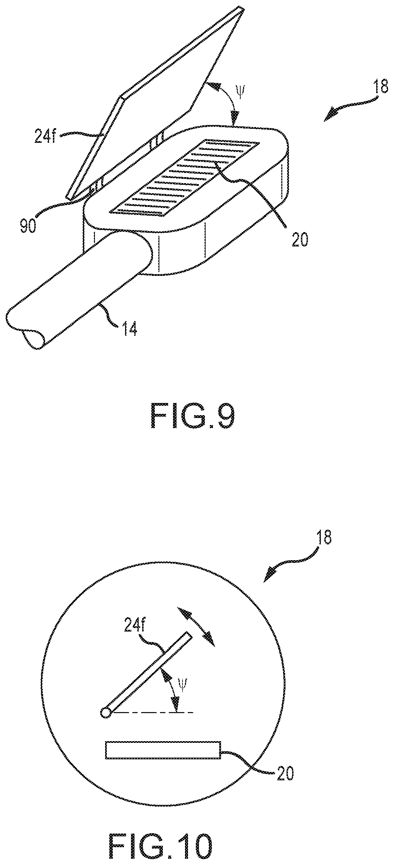

An alternative aspect of the instant teachings is illustrated in Figures 9 and 10. In this embodiment, energy deflector 24 takes the form of a reflective acoustic mirror 24f that reflects the acoustic energy impinging thereon (including both outgoing energy emitted from imaging element 20 and incoming acoustic energy returning from the tissue being imaged). Suitable materials for acoustic mirror 24f include, without limitation, stainless steel, titanium, and tungsten.

As shown in Figure 9, acoustic mirror 24f is secured to distal section 18 via hinges 90. For insertion through a patient's vasculature, acoustic mirror 24f can be stowed flat against the surface of imaging element 20. Indeed, hinges 90 can be elastic elements that bias acoustic mirror 24f into the flat, stowed position by default.

*In vivo,* acoustic mirror 24f can be deployed and caused to oscillate using any of the mechanisms described above (*e.g*., a motor, a piezomotor, a fluid driven impeller, a piezo-driven fluidic piston, a bellows, or balloon, etc.). Once deployed, and as acoustic mirror 24f oscillates, it will define an angle ψ with the face of imaging element 20. In certain aspects, acoustic mirror 24f oscillates through a total range of about 20 degrees. For example, if one assumes a "neutral" acoustic mirror 24f position of ψ = 45 degrees (that is, the position of acoustic mirror 24f corresponding to the central two-dimensional image slice), then the total oscillatory range can be defined as 35 ≤ ψ ≤ 55.

Advantageously, the volumetric range imaged by a reflective oscillating acoustic mirror 24f is twice the oscillatory range. Thus, if acoustic mirror 24f oscillates through a range of about 20 degrees total, it will be able to image a three-dimensional volume spanning about 40 degrees total (*e.g*., a total of 41 two-dimensional image slices). This yields a two-times advantage over extant mechanical wobblers, which typically move the entire transducer within the imaging device tip.

It is contemplated that acoustic mirror 24f can be a permanent part of catheter 10. For example, as shown in Figure 10, acoustic mirror 24f can be enclosed within distal section 18 in much the same fashion as the prismatic and/or lensed embodiments discussed above. Alternatively, acoustic mirror 24f can be provided as part of an external assembly designed to clip on to or slip over an extant ICE catheter (or other medical device); in this aspect, any connections necessary to acoustic mirror 24f may, for example, be routed along the outside of shaft 14.

It should also be understood, by analogy to Figures 3a-3d and their corresponding description, that acoustic mirror 24f could be modified (*e.g.,* to have a curved, rather than planar, reflective surface) to achieve analogous results.

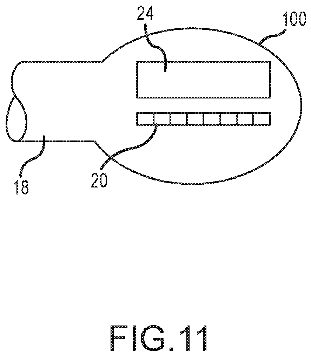

In still another embodiment, illustrated in Figure 11, imaging element 20 and energy deflector 24 are enclosed in a balloon 100 secured to distal section 18. Although Figure 11 is not drawn to scale, it should be understood that balloon 100 can be egg-shaped and have an outer diameter of about 3-4 times the diameter of catheter 10 (*e.g.,* of shaft 14). The use of balloon 100 protects the heart wall from trauma (*e.g*., hypothetical abrasion resulting from the oscillation of energy deflector 24) and minimizes or eliminates vibration of the tip of catheter 10. Balloon 100 can also be configured to drive energy deflector 24, for example by inflating balloon 100 with pumped saline and deflating balloon 100 under saline suction.

The devices disclosed herein can gather three-dimensional volumes during oscillation of energy deflector 24. It is also contemplated that the systems disclosed herein can control the oscillation of energy deflector 24 to "lock on" to a particular region of tissue, or even particular two-dimensional image slice(s). This ability to "lock on" to a target can save time (*e.g.,* a practitioner need not manually re-aim the ICE catheter periodically) and/or resources (*e.g*., it may reduce or eliminate the need for a practitioner dedicated to aiming the ICE catheter).

Although several embodiments of this invention have been described above with a certain degree of particularity, those skilled in the art could make numerous alterations to the disclosed embodiments without departing from the scope of this invention as defined in the appended claims.

For example, imaging element 20 can include one or more capacitive micromechanical ultrasound transducers ("CMUT").

As another example, the hinge or pivot about which energy deflector 24 deflects may not only rotate energy deflector 24, but also allow for translation of energy deflector 24.

As yet another example, imaging element can alternatively be coupled to a higher-power energy source, which can allow the use of imaging element for ablation as well (*e.g*., high intensity focused ultrasound ("HIFU") ablation).

All directional references (*e.g*., upper, lower, upward, downward, left, right, leftward, rightward, top, bottom, above, below, vertical, horizontal, clockwise, and counterclockwise) are only used for identification purposes to aid the reader's understanding of the present invention, and do not create limitations, particularly as to the position, orientation, or use of the invention. Joinder references (*e.g*., attached, coupled, connected, and the like) are to be construed broadly and may include intermediate members between a connection of elements and relative movement between elements. As such, joinder references do not necessarily infer that two elements are directly connected and in fixed relation to each other.

It is intended that all matter contained in the above description or shown in the accompanying drawings shall be interpreted as illustrative only and not limiting. Changes in detail or structure may be made without departing from the scope of the invention as defined in the appended claims.

## Claims

1. An apparatus for imaging tissue in three dimensions, comprising
a shaft (14),
an acoustic imaging element (20) disposed within the shaft (14) and configured to capture a two-dimensional image slice of a tissue to be imaged, the imaging element (20) including an active face that emits energy along a beam path and towards the tissue to be imaged, wherein the beam path is oriented perpendicular to a longitudinal axis of the shaft,
an energy deflector (24) positioned within the beam path, and
a drive assembly (30) coupled to the energy deflector (24), **characterised in that** the drive assembly is operable to oscillate the energy deflector (24) about the longitudinal axis of the shaft on a pivot or a hinge which is positioned running through the energy deflector (24) or on a side of the energy deflector (24).

2. The apparatus according to claim 1, further comprising a sensor for measuring a rotational position of the energy deflector (24) as it oscillates.

3. The apparatus according to claim 1 or 2, wherein the drive assembly (30) comprises a stepper motor, or
wherein the drive assembly comprises a piezomotor, or
wherein the drive assembly comprises an inflatable element, or
wherein the drive assembly comprises a fluid-driven assembly.

4. The apparatus according to any one of claims 1 to 3, further comprising a processor configured to assemble a three-dimensional volumetric image of the tissue to be imaged from a plurality of two-dimensional image slices of the tissue, wherein each image slice of the plurality of two-dimensional image slices is associated with a corresponding rotational position of the energy deflector (24).

5. The apparatus according to any one of claims 1 to 4, wherein the imaging element (20) is configured to emit energy along the beam path at a frame rate, the energy deflector (24) is configured to oscillate at an oscillation frequency, and the frame rate and the oscillation frequency are integer multiples of each other.

6. The apparatus according to claim 5, wherein the frame rate and the oscillation frequency are identical.

7. The apparatus according to any one of claims 1 to 6, wherein the energy deflector (24) is configured to oscillate at a frequency of between 15 Hz and 30 Hz.

8. The apparatus according to any one of claims 1 to 7, wherein the energy deflector (24) is configured to oscillate through a range of 70 degrees.

9. The apparatus according to any one of claims 1 to 8, further comprising an ultrasound-transmissive enclosure, and wherein at least one of the imaging element (20) and the energy deflector (24) are disposed within the enclosure.

10. The apparatus according to any one of claims 1 to 9, wherein the energy deflector (24) comprises a prism (24a).

11. The apparatus according to any one of claims 1 to 9, wherein the energy deflector (24) comprises a lens (24b).

12. The apparatus according to any one of claims 1 to 9, wherein the energy deflector (24) comprises an acoustic mirror (24f).

13. The apparatus according to claim 12, wherein the acoustic mirror (24f) is secured to the apparatus via at least one elastic element biased such that, when the elastic element is in a relaxed position, the acoustic mirror (24f) forms an angle of zero degrees with the active face of the imaging element (20).

## Patentansprüche

1. Gerät zum Aufnehmen von Gewebe in drei Dimensionen, mit
einem Schaft (14),
einem akustischen Aufnahmeelement (20), das innerhalb des Schaftes (14) angeordnet ist und dazu konfiguriert ist, einen zweidimensionalen Bildschnitt eines aufzunehmenden Gewebes aufzunehmen, bei dem das Aufnahmeelement (20) eine aktive Fläche aufweist, die Energie entlang eines Strahlungsweges und in Richtung des aufzunehmenden Gewebes emittiert, bei dem der Strahlungsweg senkrecht zu einer Längsachse des Schaftes orientiert ist,
einem Energiereflektor (24), der innerhalb des Strahlungsweges positioniert ist, und
einer Antriebsbaugruppe (30), die mit dem Energiereflektor (24) gekoppelt ist,
**dadurch gekennzeichnet, dass** die Antriebsbaugruppe zum Oszillieren des Energiereflektors (24) um die Längsachse des Schaftes um ein Gelenk oder ein Scharnier, welches derart positioniert ist, dass es durch den Energiereflektor (24) läuft oder an einer Seite des Energiereflektors (24) ist, betreibbar ist.

2. Gerät nach Anspruch 1, das ferner einen Sensor zum Messen einer Drehposition des Energiereflektors (24) während des Oszillierens aufweist.

3. Gerät nach Anspruch 1 oder 2, bei dem die Antriebsbaugruppe (30) einen Schrittmotor aufweist, oder
bei dem die Antriebsbaugruppe einen Piezomotor aufweist, oder
bei dem die Antriebsbaugruppe ein aufblasbares Element aufweist, oder
bei dem die Antriebsbaugruppe eine fluidangetriebene Baugruppe aufweist.

4. Gerät nach einem der Ansprüche 1 bis 3, das ferner einen Prozessor aufweist, der dazu konfiguriert ist, ein dreidimensionales volumetrisches Bild des aufzunehmenden Gewebes aus einer Mehrzahl von zweidimensionalen Bildschnitten des Gewebes zu bilden, bei dem jeder Bildschnitt der Mehrzahl von zweidimensionalen Bildschnitten mit einer entsprechenden Drehposition des Energiereflektors (24) assoziiert ist.

5. Gerät nach einem der Ansprüche 1 bis 4, bei dem das Aufnahmeelement (20) dazu konfiguriert ist, Energie entlang des Strahlungsweges mit einer Aufnahmefrequenz zu emittieren, der Energiereflektor (24) dazu konfiguriert ist, mit einer Oszillationsfrequenz zu oszillieren, und die Aufnahmefrequenz und die Oszillationsfrequenz ganzzahlige Vielfache zueinander sind.

6. Gerät nach Anspruch 5, bei dem die Aufnahmefrequenz und die Oszillationsfrequenz identisch sind.

7. Gerät nach einem der Ansprüche 1 bis 6, bei dem der Energiereflektor (24) dazu konfiguriert ist, mit einer Frequenz zwischen 15 Hz und 30 Hz zu oszillieren.

8. Gerät nach einem der Ansprüche 1 bis 7, bei dem der Energiereflektor (24) dazu konfiguriert ist, über einen Bereich von 70 Grad zu oszillieren.

9. Gerät nach einem der Ansprüche 1 bis 8, das ferner eine ultraschalldurchlässige Umhausung aufweist, wobei zumindest eines von dem Aufnahmeelement (20) und dem Energiereflektor (24) innerhalb der Umhausung angeordnet sind.

10. Gerät nach einem der Ansprüche 1 bis 9, bei dem der Energiereflektor (24) ein Prisma (24a) aufweist.

11. Gerät nach einem der Ansprüche 1 bis 9, bei dem der Energiereflektor (24) eine Linse (24b) aufweist.

12. Gerät nach einem der Ansprüche 1 bis 9, bei dem der Energiereflektor (24) einen akustischen Spiegel (24f) aufweist.

13. Gerät nach Anspruch 12, bei dem der akustische Spiegel (24f) an dem Gerät über zumindest ein elastisches Element gesichert ist, das derart vorgespannt ist, wenn das elastische Element in einer ausgeglichenen Position ist, der akustische Spiegel (24f) einen Winkel von null Grad mit der aktiven Fläche des Aufnahmeelementes (20) ausbildet.

## Revendications

1. Appareil d'imagerie de tissu en trois dimensions, comprenant
un arbre (14),
un élément d'imagerie acoustique (20) disposé à l'intérieur de l'arbre (14) et configuré pour capturer une tranche d'image bidimensionnelle d'un tissu à imagé, l'élément d'imagerie (20) comprenant une face active qui émet une énergie suivant un trajet de faisceau et vers le tissu à imagé, dans lequel le trajet de faisceau est orienté perpendiculairement à un axe longitudinal de l'arbre,
un déflecteur d'énergie (24) positionné à l'intérieur du trajet de faisceau, et
un ensemble d'entraînement (30) couplé au déflecteur d'énergie (24) **caractérisé en ce que** l'ensemble d'entraînement peut fonctionner pour faire osciller le déflecteur d'énergie (24) autour de l'axe longitudinal de l'arbre sur un pivot ou une articulation qui est positionné passant dans le déflecteur d'énergie (24) ou sur un côté du déflecteur d'énergie (24).

2. Appareil selon la revendication 1, comprenant en outre un capteur pour mesurer une position de rotation du déflecteur d'énergie (24) lorsqu'il oscille.

3. Appareil selon la revendication 1 ou 2, dans lequel l'ensemble d'entraînement (30) comprend un moteur pas à pas, ou
dans lequel l'ensemble d'entraînement comprend un moteur piézo-électrique, ou
dans lequel l'ensemble d'entraînement comprend un élément gonflable, ou
dans lequel l'ensemble d'entraînement comprend un ensemble entraîné par fluide.

4. Appareil selon l'une quelconque des revendications 1 à 3, comprenant en outre un processeur configuré pour assembler une image volumétrique tridimensionnelle du tissu à imager à partir d'une pluralité de tranches d'image bidimensionnelles du tissu, dans lequel chaque tranche d'image de la pluralité de tranches d'image bidimensionnelles est associée à une position rotative correspondante du déflecteur d'énergie (24).

5. Appareil selon l'une quelconque des revendications 1 à 4, dans lequel l'élément d'imagerie (20) est configuré pour émettre de l'énergie le long du trajet de faisceau à une fréquence de trame, le déflecteur d'énergie (24) est configuré pour osciller à une fréquence d'oscillation, et la fréquence de trame et la fréquence d'oscillation sont des multiples entiers l'un de l'autre.

6. Appareil selon la revendication 5, dans lequel la fréquence de trame et la fréquence d'oscillation sont identiques.

7. Appareil selon l'une quelconque des revendications 1 à 6, dans lequel le déflecteur d'énergie (24) est configuré pour osciller à une fréquence comprise entre 15 Hz et 30 Hz.

8. Appareil selon l'une quelconque des revendications 1 à 7, dans lequel le déflecteur d'énergie (24) est configuré pour osciller dans une plage de 70 degrés.

9. Appareil selon l'une quelconque des revendications 1 à 8, comprenant en outre une enceinte transmettant des ultrasons, et dans lequel au moins un élément parmi l'élément d'imagerie (20) et le déflecteur d'énergie (24) sont disposés dans l'enceinte.

10. Appareil selon l'une quelconque des revendications 1 à 9, dans lequel le déflecteur d'énergie (24) comprend un prisme (24a).

11. Appareil selon l'une quelconque des revendications 1 à 9, dans lequel le déflecteur d'énergie (24) comprend une lentille (24b).

12. Appareil selon l'une quelconque des revendications 1 à 9, dans lequel le déflecteur d'énergie (24) comprend un miroir acoustique (24f).

13. Appareil selon la revendication 12, dans lequel le miroir acoustique (24f) est fixé à l'appareil par l'intermédiaire d'au moins un élément élastique sollicité de telle sorte que, lorsque l'élément élastique est dans une position détendue, le miroir acoustique (24f) fait un angle de zéro degré avec la face active de l'élément d'imagerie (20).
